# EUROPEAN PATENT SPECIFICATION

(11) **EP 2 510 360 B1**
(45) Date of publication and mention of the grant of the patent: **22.07.2015**
(21) Application number: 10795500.7
(22) Date of filing: 30.11.2010
(51) Int. Cl.: G01N 33/68

(54) **METHOD FOR ASSESSING THE DAMAGE OF KERATIN FIBERS**
VERFAHREN ZUR BESTIMMUNG DES ZUSTANDES VON KERATINFASERN
PROCÉDÉ D'ÉVALUATION DES DÉGÂTS CAUSÉS À DES FIBRES DE KÉRATINE

(30) Priority: 07.12.2009 US 267093 P
(43) Date of publication of application: 17.10.2012
(73) Proprietor: THE PROCTER & GAMBLE COMPANY, Cincinnati, OH 45202 (US)
(72) Inventor: KUDO, Hiroyuki, Kobe Hyogo 658-0047 (JP)
(74) Representative: Boubel, Thomas
(86) International application number: PCT/US2010/058260
(87) International publication number: WO 2011/071713

(56) References cited:
- EP-A1- 1 629 775
- EP-A1- 1 696 233
- JP-A- 9 127 105
- SANDHU S S ET AL: "A SENSITIVE FLUORESCENCE TECHNIQUE USING DANSYL CHLORIDE TO ASSESS HAIR DAMAGE", JOURNAL OF THE SOCIETY COSMETIC CHEMISTS, SOCIETY OF COSMETIC CHEMISTS, US, vol. 40, no. 5, 1 September 1989 (1989-09-01), pages 287-296, XP009144064, ISSN: 0037-9832

## Description

### FIELD OF THE INVENTION

In a first aspect, the present invention relates to a method for assessing damages of keratin fibers using a cationic fluorescent compound comprising a cationic ammonium group and being free of carboxyl and/or sulfonyl groups. In a second aspect, the present invention relates to a method for comparing the damages of different keratin fibers using said cationic fluorescent compound. Said methods are useful for quantitatively and/or qualitatively assessing the degree of damages of keratin fibers and also to compare the damages of fibers of different origin, different portions of fibers and/or fibers treated with different cosmetic, chemical and/or mechanical treatments. Said methods are also useful for supporting advertising claims about the superiority of a composition and/or a treatment versus others.

### BACKGROUND OF THE INVENTION

Keratin fibers, particularly human hair fibers, may be damaged over time. Damages may concern the outer layer of the fiber (i.e. the cuticle) and/or the inner layers (i.e. the cortex and the medulla). The cuticle consists of flat overlapping cells (scales), which are attached at the proximal end (root end), and they point toward the distal end (tip end) of the hair fiber. When having no surface damages, the cuticle usually comprises smooth undamaged scale edges and scale surfaces. In contrast, when hair fibers are damaged, scale edges are damaged. Damages, particularly those to the cuticle, make hair fibers more vulnerable to chemical and mechanical breakdown and impact on the behavior and the cosmetic behavior of hair, including the elastic and tensile deformations, the bending and fiber stiffness, and torsion and fiber rigidity.

Damages may be caused by environmental factors, including air pollution, sun exposure, water pool, and/or rain. Damages may also be caused by applying to the fibers grooming (cosmetic), chemical and/or mechanical treatments. Grooming treatments, particularly shampoo, are conventionally used in order to cleanse the hair. However, shampoos may damage the hair in different ways. They may damage the hair by abrasion and/or erosion, and also by slowly dissolving or removing structural lipids and proteinaceous material from hair. Chemical treatments usually involve modifying the structure of hair and may impart severe damages to hair fibers. This includes for example reducing hair (such as permanent waving processes), bleaching hair and/or dyeing hair using oxidation dyes and/or permanent hair dyes. Mechanical treatments, particularly brushing, combing, towel drying and blow-drying, may damage the hair by abrasion and/or erosion.

Damages may be prevented and/or at least partially repaired by applying a suitable treatment. Particularly, conditioning compositions are conventionally used to prevent and/or repair damages. They may comprise cationic surfactant in combination with long chain fatty alcohol and/or other lipid compounds. They may also comprise silicone-based compounds.

Assessing the degree of damages caused to hair is of interest in order to understand the impact of various environmental factors as well as the impact of the cosmetic (grooming), chemical and mechanical treatments onto keratin fibers. Such assessment is also of interest in order to demonstrate the efficacy of treatments used for preventing and/or repairing hair damages. Several attempts for assessing hair damages, using different analytical methods, have already been reported. However, such methods usually require using complex and expensive technical devices and/or do not provide reliable data. Particularly, it has already been reported methods using some fluorescent compounds. However, when comparing for example healthy hair (i.e. undamaged) and damaged hair, the data obtained between both hairs seems to be not significantly different.

There is a need therefore for providing a method for assessing damages of keratin fibers, particularly, human hair fibers. Particularly, there is a need for providing a method for assessing damages of treated keratin fibers using cosmetic, chemical and/or mechanical treatments. In addition, there is also a need for providing a method for assessing and comparing damages of different keratin fibers, e.g. untreated fibers versus treated fibers or fibers treated with different treatments. As far as cosmetic compositions - particularly conditioning compositions - are concerned, there is a need for providing a method for assessing the efficacy of such compositions for preventing and/or repairing damages of hair fibers. There is also a need for providing a method for comparing the efficacy of two or more cosmetic compositions, particularly conditioning compositions, for preventing and/or treating damages of hair fibers. Finally, there is a need for providing a method for supporting advertising claims about the efficacy of a composition - or about the superiority of this composition versus a comparative composition - for preventing and/or repairing damages of hair fibers.

### SUMMARY OF THE INVENTION

The present invention relates to a method for assessing damages of keratin fibers comprising particularly the steps of tagging at least one sample of fiber(s) with a specific cationic fluorescent compound and assessing the fluorescence of the tagged fibers. The present invention also relates to a method for assessing and comparing damages of keratin fibers particularly comprising the steps of providing at least two different samples of fiber(s), tagging them with a specific cationic fluorescent compound, and assessing and then comparing the fluorescence of the tagged fibers. These methods allow assessing and comparing the degree of damages of fibers after exposure to environmental exposure and after treatments using cosmetic, chemical and/or mechanical treatments. These methods also allow assessing the efficacy of composition(s) for preventing and/or repairing damages to hair. Furthermore, these methods allow assessing and comparing the efficacy of compositions for preventing and/or repairing damages to hair.

### DETAILED DESCRIPTION OF THE INVENTION

In a first aspect, the present invention relates to a method for assessing damages of keratin fibers comprising the steps of:
- providing at least one sample of keratin fiber(s);
- providing a cationic fluorescent compound comprising a quaternary ammonium group and being free of carboxyl and/or sulfonyl groups;
- tagging said sample of fiber(s) with said cationic fluorescent compound;
- assessing the fluorescence of the tagged fiber(s) using a source providing appropriate excitation wavelength(s).

The inventors have surprisingly found that the degree of keratin fiber-surface damages may be assessed, both qualitatively and quantitatively, by using fluorescence microscopy and by carefully selecting the fluorescent compound to be used. Indeed, the inventors have found that cationic fluorescent compounds comprising at least one quaternary ammonium group and being free of carboxyl and/or sulfonyl groups allow, when used in combination with a device emitting an appropriate excitation wavelength, the obtaining of data suitable for assessing the fluorescence of the fibers and correlating such fluorescence with a certain degree of damages. Likewise, these specific cationic fluorescent compounds allow the obtaining of data suitable for comparing the fluorescence of the treated fibers versus untreated fibers, or fibers treated with two different treatments. Without wishing to be bound by theory, it is believed that cationic fluorescent compounds comprising at least one quaternary ammonium group and being free of carboxyl and/or sulfonyl groups interact significantly more with damaged fibers versus undamaged fibers and that such interaction increases with the degree of damages of the fibers. Particularly, it is believed that these cationic fluorescent compounds comprising at least one quaternary ammonium group and being free of carboxyl and/or sulfonyl groups interact specifically with damaged scale edges and surfaces (typical of damaged fibers), particularly by means of electrostatic attraction. In contrast, they interact significantly less, if not at all, with smooth undamaged scale edges and scale surfaces (typical of healthy or undamaged fibers).

The method, according to the invention, comprises the provision of at least one sample of keratin fiber(s) (so-called "provision step"). Said sample may comprise one fiber, two or several fibers. When said sample comprises at least two fibers, said fibers may be bundled to each other at one end, or alternatively at both ends. Said sample usually comprises fibers of the same origin (e.g. from the same person and the same region of the body), and/or of the same portion (e.g. root end or tip end of hair fibers), and/or having been subjected to the same cosmetic, chemical and/or mechanical treatments.

The fibers may be of sufficient length for being put in contact with the cationic fluorescent compound and for having their fluorescence assessed. The fibers have preferably a length of 1 cm to 10 cm, more preferably of 1 cm to 5 cm, still more preferably of 2 cm to 5 cm.

The fibers may be selected from mammal hair, preferably from human hair, more preferably from human female hair. The origin of the human hair may be Caucasian, African, Asian, or any other origin.

The hair fiber may be obtained from any part of the body, e.g. the legs, the arms, the torso, the face or the scalp. The hair fiber is preferably obtained from the scalp.

The method further comprises the step of providing a cationic fluorescent compound comprising at least one quaternary ammonium group and being free of carboxyl and/or sulfonyl groups (so-called "provision step"). This compound is capable of interacting with negatively charged keratin fiber(s). This compound may also be called cationic fluorescent probe. Particularly, said interaction may occur by means of electrostatic attraction between the positively charged quaternary ammonium groups of the cationic fluorescent compounds and the negatively charged groups of the keratin fiber(s). The inventors have surprisingly found that the interaction between the cationic fluorescent compounds and the hair fibers (and therefore the fluorescence intensity) is closely related with the overall negative charge of keratin fibers, which is associated with the degree of damage. Using these specific cationic fluorescent compounds allow therefore demonstrating a clear correlation between the fluorescence intensity and the degree of damages of keratin fibers. In contrast, the inventors have also found that, when using a cationic fluorescent compound comprising at least one carboxyl and/or sulfonyl group, a large variation of the fluorescence intensity is obtained, particularly when working with undamaged hair fibers. Such compound is for example Ethanaminium, N-[9-(2-Carboxyphenyl)-6-(Diethylamino)-3H-Xanthen-3-ylidene]-N-Ethyl-, Chloride. Such variation does not render difficult the correlation between the fluorescence intensity and the degree of damages.

The cationic fluorescent compound may be selected from the group of cationic fluorescent compounds comprising at least one quaternary ammonium group and being free of carboxylic group and/or sulfonyl groups and belonging to the classes of acridine, azo, diarylmethane, eurhodin, oxazone, thiazole, triarylmethane, safranin, or mixtures thereof.

Particularly, the cationic fluorescent compound may be selected from the group of compounds of Colour Index (referred hereinafter as "C.I.") number 46005, 41000, 50040, 51180, 49005, 42000, 42040, 42500, 42510, 42520, 42555, 42563, 42585, 42600, 44045, 44085, 50240, or mixtures thereof. Said compounds may be further defined by their common names, C.I. names and/or empirical formula as follows 46005 (Acridine orange, basic orange 14, C₁₇H₂₀N₃Cl), 41000 (Auramine O, basic yellow 2, C₁₇H₂₂N₃), 50040 (Neutral red, basic red 5, C₁₅H₁₇N₄Cl), 51180 (Nile blue, basic blue 12, C₂₀H₁₈N₂O₂), 49005 (Thioflavine T, basic yellow 1, C₁₇H₁₉N₂SCl), 42000 (Malachite green, basic green 4, C₂₃H₂₅N₂Cl), 42040 (Brilliant green, basic green 1, C₂₇H₃₄N₂O₄S), 42500 (Pararosanilin, basic red 9, C₁₉H₁₈N₃Cl), 42510 (Rosanilin, basic violet 14, C₂₀H₂₀N₃Cl), 42520 (New Fuschin, basic violet 2, C₂₂H₂₄N₃Cl), 42555 (Crystal violet, basic violet 3, C₂₅H₃₀N₃Cl), 42563 (Victoria blue, basic blue 8, C₃₄H₃₄N₃Cl), 42585 (Methyl green, basic blue 20, C₂₆H₃₃N₃Cl₂), 42600 (Ethyl violet, basic violet 4, C₃₁H₄₂N₃Cl), 44045 (Victoria blue, basic blue 26, C₃₃H₃₂N₃Cl), 44085 (Night blue, basic blue 15, C₃₈H₄₂N₃Cl), 50240 (Safranin, basic red 2, C₂₀H₁₉N₄Cl (dimethyl) and C₂₁H₂₁N₄Cl (trimethyl)). The cationic fluorescent compound may be any alternative compound as far as this compound comprises at least one quaternary ammonium group, is free of carboxyl and/or sulfonyl groups and is capable of interacting with negatively charged keratin fiber(s).

The cationic fluorescent compound may be obtained by quaternization of a cationic fluorescent compound comprising a primary, a secondary and/or a tertiary ammonium group using any suitable alkylating agent such as methyl iodide. For example, the cationic fluorescent compound may be obtained from a compound selected from the group of compounds of C.I. number (common name, C.I. name and/or empirical formula) 21000 (Bismarck brown, basic brown 1, C₁₈H₂₀N₈Cl₂), 52015 (Methylene blue, basic blue 9, C₁₆H₁₈N₃SCl), 52020 (Methylene green, basic green 5, C₁₆H₁₇NO₂SCl), 52020 (Toluidine blue, basic blue 17, C₁₅H₁₆N₃SCl).

The cationic fluorescent compound is preferably selected from the group of compounds belonging to the class of thiazole. In a preferred embodiment, the cationic fluorescent compound is a thiazole compound of formula I

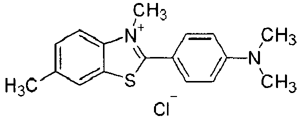

Said thiazole compound of formula I is conventionally known, and hereinafter referred to as, Thioflavine T. Thioflavine T has a molecular weight of 318.9 and emits fluorescence from 450 nm to 600 nm of emission wavelength at 400 nm to 440 nm of excitation wavelength. Particularly, Thioflavine T emits a green fluorescence from 500 nm to 560 nm of emission wavelength. A commercially available source of said thiazole compound of formula I is for example Thioflavine T from Sigma. Providing Thioflavine T is particularly beneficial as it has been found by the inventors that the interaction between this compound and the keratin fibers differs significantly depending on whether the keratin fibers are damaged or not, and on the degree of damage. Thioflavine T is particularly suitable therefore for comparing the degree of damages between keratin fibers of different origin, from different portion and/or having been subjected to different treatments.

The cationic fluorescent compound may be provided in any appropriate form, e.g. pulverulent, liquid or solid form. The cationic fluorescent compound is provided preferably in the form of a solution, more preferably in the form of an aqueous solution. The solution may comprise from 1 ppm to 3000 ppm, preferably from 100 ppm to 1000 ppm, more preferably from 400 ppm to 600 ppm, of said compound by total weight of the composition. The solution may also comprise a suitable aqueous carrier, preferably water.

The method further comprises the step of tagging the sample(s) of fiber(s) with the cationic fluorescent compound (so-called "tagging step"). The tagging step may be carried out by putting the sample(s) in contact with the cationic fluorescent compound by any suitable means and for a defined period of time sufficient for the cationic fluorescent compound to interact with the fiber(s). After carrying out this step, the fiber(s) may be referred hereinafter as "tagged fiber(s)".

When the cationic fluorescent compound is provided in the form of a solution, the sample(s) is(are) put in contact with the cationic fluorescent compound by means of immersing said sample(s) into the solution.

In order to ensure that the cationic fluorescent compound has sufficient time for interacting with the fiber(s), the fiber(s) is(are) preferably put in contact with the cationic fluorescent compound for 1 sec to 10 min, more preferably 1 sec to 1 min.

The method further comprises the step of assessing the fluorescence of the tagged fiber(s) using a source providing appropriate excitation wavelength(s) (so-called "assessment step"). The excitation wavelengths would be dependent on the cationic fluorescent compound used. Indeed, each cationic fluorescent compound has a specific absorption spectrum. In order to obtain the compound in an excited state (so that the compound would emit light at specific wavelengths, referred hereinafter to as "emission wavelength"), the source should provide appropriate excitation wavelengths. Any commercially available source providing specific wavelengths may be used.

Thioflavine T has a maximum absorption at 440 nm and emits - when in an excited state - "fluorescent" emission wavelengths from 450 nm to 600 nm (particularly from 500 nm to 560 nm for green fluorescence). In order to excite Thioflavine T, excitation wavelengths of 400 nm to 440 nm should be provided.

The step of assessing the fluorescence of the tagged fiber(s) may be carried out using any device suitable for detecting, and if needed displaying and/or recording, the wavelengths emitted (i.e. the fluorescence) by the cationic fluorescent compound. The detection may be carried out using a fluorescence microscope. A commercially available fluorescence microscope is for example Eclipse 80i from Nikon or Cyscope from Partec. The assessment of the fluorescence may be assisted using suitable software(s). Commercially available software for observation is for example DynamicEye REAL from Mitani Corporation and LuminaVision from Mitani Corporation. Commercially available software for image analysis is for example LuminaVision from Mitani Corporation.

The fluorescence of the sample(s) may be assessed qualitatively by visual inspection, either by direct visual inspection such as observation through the binocular or by indirect visual inspection such as picture analysis. The fluorescence of the sample(s) may also be assessed quantitatively by data analysis.

The method may also comprise the step of treating the sample(s) of fiber(s) (so-called "treatment step"). The treatment step is preferably carried out after the provision step and before the tagging step. The treatment step may be carried out by treating the sample(s) of fiber(s) using any suitable cosmetic composition, chemical and/or mechanical treatment(s).

This step may be carried out by applying a cosmetic composition onto keratin fiber(s). Any suitable cosmetic composition known in the art may be used such as shampoo(s), conditioning composition(s), hair rinse-off treatment(s), hair leave-on treatment(s), styling composition(s). For example, any commercially available shampoos, conditioners, hair rinse-off treatments and hair leave-on treatments of tradename Pantene® and Head & Shoulders® may be used.

Only one composition may be applied onto fiber(s). Alternatively, two or several compositions may be applied simultaneously or sequentially. In addition, before and/or after applying each composition, the fiber(s) may further be wetted, rinsed and/or dried. In one embodiment, the treating step comprises treating (washing) the fiber(s) with a shampoo, then rinsing the washed fiber(s) with water, then drying the fiber(s). In another embodiment, the treating step comprises treating (washing) the fiber(s) with a shampoo, then rinsing the washed fiber(s) with water, then treating the fiber(s) with a conditioning composition, then rinsing the treated fiber(s) with water, then drying the fiber(s). In another embodiment, the treating step comprises treating (washing) the fiber(s) with a shampoo, then rinsing the washed fiber(s) with water, then treating the fiber(s) with a conditioning composition, then rinsing the treated fiber(s) with water, then treating at least one time the fiber(s) with a hair rinse-off treatment, then rinsing the treated fiber(s) with water, then drying the fiber(s).

Alternatively or complementary, this step may be carried out by chemically treating the sample(s) of fiber(s) using a chemical treatment. Any suitable chemical treatment known in the art may be used such as permanent waving treatment, bleaching treatment and/or color-dyeing treatment.

Alternatively or complementary, this step may be carried out by mechanically treating the sample(s) of fiber(s). Any suitable mechanical treatment known in the art may be used such as brushing, combing, towel rubbing, and/or blow drying.

The method may also comprise the step of rinsing out the excess of cationic fluorescent compound (so-called rinsing step). The rinsing step may be carried out after the tagging step and before the assessment step. Rinsing the fiber(s) in order to remove the excess of cationic fluorescent compound - i.e. compounds not interacting with the fiber(s) (co-called "free compounds") - is beneficial for avoiding the assessment of the fluorescence of the fibers to be impacted by the presence of free compounds. The fibers may be rinsed using water. Particularly, this step may be carried out by immersing the sample(s) of fiber(s) in an aqueous solution for a defined period of time sufficient for removing the excess of cationic fluorescent compound. The aqueous solution is preferably water, more preferably deionised water. The period of time is preferably from 1 sec to 10 min, more preferably from 1 sec to 1 min.

The method may also comprise the step of utilizing said assessment to support advertising claims (so-called "advertising step"). Indeed, the present method allows correlating the degree of damages of keratin fibers with the fluorescence intensity. When advertising one treatment (e.g. a conditioning composition), the data and/or the pictures obtained using this method may be used therefore support and/or demonstrate advertising claims according to which said treatment prevent and/or repair fiber damages.

In a second aspect, the present invention relates to the method comprising the steps of:
- providing at least two different samples of keratin fiber(s);
- providing a cationic fluorescent compound comprising at least one quaternary ammonium group and being free of carboxyl and/or sulfonyl groups;
- tagging said samples of fiber(s) with said cationic fluorescent compound;
- assessing the fluorescence of each sample of fiber(s) using a source providing appropriate excitation wavelength(s); and,
- comparing the fluorescence between the samples.

The method comprises the step of providing at least two, preferably from two to four, more preferably two, different samples of keratin fiber(s). As used herein, "different samples" means samples differing from each other by the origin of the fibers, the portion of the fibers and/or the treatment(s) applied to fibers.

In one embodiment, the samples may be obtained from a different person. Alternatively, the samples may be obtained from the same person but from a different part of the body.

In another embodiment, one sample may comprise the tip portion of fiber(s) and the other sample may comprise the root portion of the same fiber(s). Providing and comparing different portions of the same fiber(s), particularly tip versus root, allows assessing the difference of the degree of damages over time, as the fiber(s) grows(-).

In another embodiment, one sample may comprise untreated fiber(s) and the other sample comprises fiber(s) treated with a cosmetic composition. The other sample may be treated with a shampoo, and/or a conditioning composition, and/or a hair rinse-off treatment, and/or a leave-on treatment, and/or any other suitable cosmetic composition. Comparing treated fiber(s) and untreated fiber(s) is beneficial for assessing the damaging effects of the compositions such as shampoos onto hair or, in contrast, for assessing the benefits of the compositions such as conditioning compositions for preventing and/or repairing the damages of the fiber(s).

In another embodiment, the samples may be treated with different cosmetic compositions. For example and non-exhaustively, (1) one sample may be treated with one shampoo and the other sample with another shampoo; (2) one sample may be treated with one shampoo and the other sample may be treated with the same shampoo and then one conditioner; (3) one sample may be treated with one shampoo and then one conditioner and the other sample may be treated with the same shampoo and then another conditioner, (4) one sample may be treated with one shampoo and then one conditioner and the other sample may be treated with the same shampoo, then the same conditioner, and then a rinse-off treatment, (5) one sample may be treated with one shampoo and then one conditioner and the other sample may be treated with the same shampoo, then the same conditioner, and then a leave-on treatment, (6) one sample may be treated with one shampoo and then one conditioner and the other sample may be treated with another shampoo and then the same conditioner; (7) one sample may be treated one time with one shampoo and the other sample may be treated two or several time with the same shampoo; (8) one sample may be treated one time with one conditioner and the other sample may be treated two or several times with the same conditioner. Comparing differently treated fiber(s) is beneficial for comparing the damaging effects of different shampoos (see (1)); for assessing the mitigating effects of conditioners onto shampoo treatments (see (2)); for comparing the benefits of conditioning compositions for preventing and/or repairing the damages of the fiber(s) (see (3)); for assessing the mitigating effects of rinse-off treatments onto shampoo treatments (see (4)); for assessing the mitigating effects of leave-on treatments onto shampoo treatments (see (5)); for comparing the mitigating effects of one conditioners onto different shampoo treatments (see (6)); for comparing the effects of repeating treatments onto fiber(s) (see (7) and (8)).

In another embodiment, one sample may comprise untreated fiber(s) and the other sample comprises fibers being chemically-treated. Alternatively, the samples may comprise fiber(s) be treated with different chemical treatments.

In another embodiment, one sample may comprise untreated fiber(s) and the other sample comprises fibers being mechanically-treated. Alternatively, the samples may comprise fiber(s) be treated with different mechanical treatments.

The provision (cationic fluorescent compound) step(s), the optional/alternative treatment step(s), the tagging step(s), the optional rinsing step(s), the assessment step, and the optional advertising step are defined hereinbefore together with the first aspect of the present invention.

The method also comprises the step of comparing the fluorescence of the samples (so-called "comparison step"). The fluorescence of the sample(s) may be compared qualitatively by visual inspection, either by direct visual inspection such as observation through the binocular or by indirect visual inspection such as picture analysis. The fluorescence of the sample(s) may also be compared quantitatively by data analysis. The comparison step is beneficial for example for comparing the effects of one treatment onto fibers (versus no treatment), for comparing the damaging effects of at least two different treatments, for comparing the efficacy of at least two different treatments for preventing and/or repairing the damages of fibers.

The method may also comprise the step of utilizing said assessment and comparison to support advertising claims (so-called "advertising step"). Making advertising steps based on the outcome of the comparison between two different samples is beneficial for example for advertising the efficacy of a treatment for preventing and/or repairing damages to fibers and/or for advertising the superiority of one treatment versus another treatment for preventing and/or repairing damages. When advertising one treatment (e.g. a conditioning composition) versus another one, the data and/or the pictures obtained using this method may be used therefore support and/or demonstrate advertising claims according to which said treatment provide higher performance versus the other one for preventing and/or repairing fiber damages.

In a specific embodiment, the present invention relates to a method for assessing damages of keratin fibers comprising the steps of:
- providing at least one sample of keratin fiber(s);
- treating said sample with a cosmetic, chemical and/or mechanical treatment(s);
- providing a cationic fluorescent compound comprising at least one quaternary ammonium group and being free of carboxyl and/or sulfonyl groups;
- rinsing out the excess of cationic fluorescent compound;
- tagging said treated sample with said cationic fluorescent compound;
- assessing the fluorescence of the tagged treated sample using a source providing appropriate excitation wavelength(s);
- optionally, using said assessment to support advertising claims about the efficacy and/or performance of the treatment for preventing and/or repairing fiber damages.

In another specific embodiment, the present invention relates to a method for assessing and comparing damages of different keratin fibers comprising the steps of:
- providing two different samples of keratin fiber(s);
- treating one of the samples with a cosmetic, chemical and/or mechanical treatment(s);
- providing a cationic fluorescent compound comprising at least one quaternary ammonium group and being free of carboxyl and/or sulfonyl groups;
- rinsing out the excess of cationic fluorescent compound;
- tagging the untreated sample and the treated sample with said cationic fluorescent compound;
- assessing the fluorescence of the tagged, untreated sample (control) and the tagged, treated sample using a source providing appropriate excitation wavelength(s);
- comparing the fluorescence of both samples;
- optionally, using said assessment and comparison to support advertising claims about the performance and/or the efficacy of the treatment versus control for preventing and/or repairing fiber damages.

In another specific embodiment, the present invention relates to a method for assessing and comparing damages of different keratin fibers comprising the steps of:
- providing two different samples of keratin fiber(s);
- treating both samples with a different cosmetic, chemical and/or mechanical treatment(s);
- providing a cationic fluorescent compound comprising at least one quaternary ammonium group and being free of carboxyl and/or sulfonyl groups;
- rinsing out the excess of cationic fluorescent compound;
- tagging both treated samples with said cationic fluorescent compound;
- assessing the fluorescence of both tagged, treated samples using a source providing appropriate excitation wavelength(s);
- comparing the fluorescence of both samples;
- optionally, using said assessment and comparison to support advertising claims about the superiority of the first treatment versus the second one for preventing and/or repairing fiber damages.

### Example

### Materials

Hair fibers: 25 cm long-Asian female hair fibers
Cationic fluorescent compound: Thioflavin T from Sigma
Microscope: Eclipse 80i from Nikon
Software: DynamicEye REAL and LuminaVision from Mitani Corporation.

Cleansing shampoo: composition of pH = 5-7 comprising ammonium AE3 sulfate (51.2%), ammonium lauryl sulfate (36.4%), cocamide DEA (2.3%), ammonium xylenesulfonate (3.5%), EDTA (0.1%), preservatives (<1%), water q.s. to 100%.

Shampoo: composition of pH = 5-7 comprising ammonium laureth sulfate (8.5%), ammonium lauryl sulfate (1.7%), cocamidopropyl betaine (1.7%), sodium lauroamphoacetate (1.7%), glycol distearate (1.5%), cocamide MEA (0.8%), fatty alcohol (0.6%), dimethicone (0.6%), polyquaternium-10 (0.4%), hydrogenated polydecene (0.2%), pro-vitamins (<0.1%), amino-acids (<0.1%), preservatives (0.4%), pro-vitamins (<0.1%), water q.s. to 100%.

Conditioning composition: composition of pH = 5-7 comprising behentrimonium methosulfate/isopropyl alcohol (2.3%), fatty alcohols (5.2%), terminal aminodimethicone (4.0%), preservatives (0.6%), water q.s. to 100%.

Rinse-off treatment: composition of pH = 5-7 comprising behentrimonium chloride (2.8%), terminal aminodimethicone (2.0%), fatty alcohols (6.5%), benzyl alcohol (0.4%), EDTA (0.1%), pro-vitamins (<0.1%), amino-acids (<0.1%), preservatives (<0.1%), water q.s. to 100%.

Percentages of compounds are weight percent per total weight of the composition.

### Protocol

Depending on the method carried out, some of the steps may be omitted, e.g. the treating step.
1. [Provision step] One sample being either the root portion of one hair fiber ("3 cm to 6 cm"-portion from hair root) or a sample of the tip portion of one hair fiber ("14 cm to 20 cm"-portion from hair root) is provided. Each sample is washed with cleansing shampoo solution (10%).
2. [Provision step] It is provided an aqueous solution comprising 500 ppm Thioflavin T (Sigma) - hereinafter referred to as "Thioflavin T solution".
3. [Treatment step] The samples are treated by applying a shampoo, then a conditioning composition, then a rinse-off treatment as described below. These samples are hereinafter referred to as "treated samples".
3.1 Each sample is completely immersed for 2 sec in a 50% shampoo solution. The sample is then removed and rubbed ten strokes from root to tip. The sample is then rinsed with deionized water for 10 sec.
3.2 Each sample is completely immersed for 2 sec in a 100% conditioning composition. The sample is then removed and rubbed two strokes from root to tip. The sample is then rinsed with deionized water for 1 sec.
3.3 Each sample is completely immersed for 2 sec in a 100% rinse-off treatment. The sample is then removed and rubbed two strokes from root to tip. The sample is then rinsed with deionized water for 1 sec.
3.4 Each treated sample is dried overnight at ambient temperature.
4. [Tagging step] Each treated sample is immersed completely in Thioflavin T solution for 10 sec.
5. [Rinsing step] Each treated and tagged sample is then immersed completely and under agitation (i.e. shaking) in dionized water for 10 sec, in order to remove excess cationic fluorescent compound (i.e. free compounds).
6. [Assessment step] Each treated, tagged sample is fixed on glass side with double-sided adhesive tape. The assessment of the fluorescence is carried out by capturing fluorescence image and normal image at reflection mode using the parameters detailed in the table I below. The green fluorescence intensity on the entire hair surface in the field of view and the fluorescence data are reported/recorded.

**Table I - Parameters and calibration**

| | |
|---|---|
| Fluorescent Block | Nikon BV-2A [Ex.400-440, DM.455, BA.470] |
| Dark Filter | NCB11, ND4 |
| Shutter Speed (sec) | 1/125, 1/180, 1/500 |
| | *Since the brightness is changed day by day due to software trouble, shutter speed is adjusted on each measured date. |

### Assessment and comparison of damages of tip portion of hair fibers versus root portion of hair fibers

Two samples (i.e. tip portion and root portion) of the same fibers are provided. The samples are left untreated. The fluorescence is assessed according to the protocol described above, except for step 3 which is omitted.

**Table II: Fluorescence intensity versus control (average of green fluorescence intensity on entire hair surface, n=40)**

| Samples | Fluorescent intensity and variation |
|---|---|
| Untreated tip portion | Control |
| Untreated root portion | 50% reduced intensity was observed compared to Control |

As shown by the data provided in table II, the fluorescence emitted by the untreated tip portion is significantly higher than the untreated root portion (with paired t-test @95% confidence level). This demonstrates that the method according to the present invention is suitable for correlating the intensity of the fluorescence of hair fibers (via the cationic fluorescent compound interacting with the fibers) with their degree of damages. The more damaged the hair fibers are, the more intense the fluorescence.

### Assessment and comparison of damages of treated versus untreated tip portions of hair fibers and differently-treated tip portions of hair

2 groups of 2 samples of the same fibers are provided. Each group comprises a treated tip portion versus untreated tip portion (controls 1 and 2). The fluorescence intensity is assessed according to the protocol described above. For controls 1 and 2, steps 3 are omitted. For the treated tip portions, step 3 is conducted as described in the table II below.

**Table III: Treatment step of the treated tip portions**

| Treatments | Groups | |
|---|---|---|
| | 1 | 2 |
| Shampoo | X | X |
| Conditioner | X | X |
| Rinse-off treatment | | X |

**Table IV: Fluorescence intensity (versus individual corresponding untreated hair tip at 100%)**

| Samples | Groups (fluorescent intensity and variation) | |
|---|---|---|
| | 1 | 2 |
| Untreated tip portion | Control 1 | Control 2 |
| Treated tip portion | 36% reduced intensity was observed compared to Control 1 | 47% reduced intensity was observed compared to Control 2 |

As shown by the data provided in table IV, the fluorescence intensity of treated tip portions is significantly lower than the fluorescence intensity of the untreated tip portions (with paired t-test @95% confidence level). This shows that the degree of damages of treated damaged fibers (herein tip portion) is significantly reduced versus untreated damaged fibers. This demonstrates that the method according to the present invention is suitable for assessing the efficacy of conditioning compositions and/or rinse-off treatments for repairing - and by extension preventing - damages to hair fibers.

As also shown by the data provided in table IV, the fluorescence intensity of treated tip portions of group 2 is significantly lower than the fluorescence intensity of treated tip portions of group 1 (with paired t-test @80% confidence level). This shows that the treatments of group 2 (including additional treatment by Rinse-off treatment) reduces the degree of damages of damaged fibers further than the treatments of group 1. This demonstrates that the method according to the present invention is suitable for assessing and comparing the efficacy of two different treatments for repairing - and by extension preventing - damages to hair fibers.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 min" is intended to mean "about 40 mm."

While particular embodiments of the present invention have been illustrated and described, it would be obvious to those skilled in the art that various other changes and modifications can be made. It is therefore intended to cover in the appended claims all such changes and modifications that are within the scope of this invention.

## Claims

1. A method for assessing damages of keratin fibers comprising the steps of:
- providing at least one sample of keratin fiber(s);
- providing a cationic fluorescent compound comprising at least one quaternary ammonium group and being free of carboxyl and/or sulfonyl groups;
- tagging said sample of fiber(s) with said cationic fluorescent compound;
- assessing the fluorescence of the fiber(s) using a source providing appropriate excitation wavelength(s).

2. A method, according to claim 1, for assessing and comparing damages of different keratin fibers comprising the steps of:
- providing at least two different samples of keratin fiber(s);
- providing a cationic fluorescent compound comprising at least one quaternary ammonium group and being free of carboxyl and/or sulfonyl groups;
- tagging said samples of fiber(s) with said cationic fluorescent compound;
- assessing the fluorescence of each sample of fiber(s) using a source providing appropriate excitation wavelength(s); and,
- comparing the fluorescence of the samples.

3. A method, according to any preceding claims, wherein the fibers are selected from mammal hair; preferably human hair; more preferably human female hair.

4. A method, according to any preceding claims, wherein the cationic fluorescent compound is selected from the group of compounds belonging to the classes of acridine, azo, diarylmethane, eurhodin, oxazone, thiazole, triarylmethane, safranin, or mixtures thereof, preferably from the group of compounds belonging to the class of thiazole.

5. A method, according to claim 4, wherein the cationic fluorescent compound is a thiazole compound of formula I:

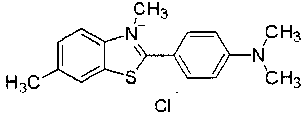

6. A method, according to according to any preceding claims, wherein the cationic fluorescent compound is provided in the form of an aqueous solution comprising from 1 ppm to 3000 ppm of said compound by total weight of the composition and an aqueous carrier.

7. A method, according to any preceding claims, wherein the fluorescence of the fiber(s) is assessed using a fluorescence microscope.

8. A method, according to claim 7, wherein the fluorescence of the sample is qualitatively assessed by visual inspection and/or wherein the fluorescence of the samples is quantitatively assessed using a software-implemented device.

9. A method, according to any preceding claims, further comprising the step of treating the sample(s) of fiber(s) using a cosmetic, chemical and/or mechanical treatment.

10. A method, according to any of claims 2 to 9, wherein the samples differ from each other by the origin of the fibers, the portion of the fibers and/or the treatment(s) applied to the fibers.

11. A method, according to claim 10, wherein one sample comprises untreated fiber(s) and the other sample comprises fiber(s) treated with a cosmetic, chemical and/or mechanical composition(s).

12. A method, according to claim 10, wherein the samples are treated with different cosmetic, chemical, and/or mechanical treatment(s).

13. A method, according to any claims 9 to 12, wherein the cosmetic treatment is selected from the group of a shampoo, a conditioning composition, a rinse-off treatment, a leave-on treatment or combinations thereof.

14. A method, according to any preceding claims, further comprising the step of rinsing out the excess of cationic fluorescent compound.

15. A method, according to any preceding claims, further comprising the step of utilizing said assessment and/or comparison to support advertising claims about the efficacy of a treatment versus a control and/or about the superiority of one treatment versus the other.

## Patentansprüche

1. Verfahren zum Bewerten von Schädigungen von Keratinfasern, umfassend die folgenden Schritte:
- Bereitstellen mindestens einer Probe einer Keratinfaser bzw. von Keratinfasern;
- Bereitstellen einer kationischen fluoreszierenden Verbindung, die mindestens eine quartäre Ammoniumgruppe umfasst und frei von Carboxyl- und/oder Sulfonylgruppen ist;
- Markieren der Probe einer Faser bzw. von Fasern mit der kationischen fluoreszierenden Verbindung;
- Bewerten der Fluoreszenz der Faser bzw. Fasern mittels einer Quelle, die eine geeignete Anregungswellenlänge bzw. geeignete Anregungswellenlängen bereitstellt.

2. Verfahren nach Anspruch 1 zum Bewerten und Vergleichen von Beschädigungen verschiedener Keratinfasern, umfassend die folgenden Schritte:
- Bereitstellen von mindestens zwei verschiedenen Proben einer Keratinfaser bzw. von Keratinfasern;
- Bereitstellen einer kationischen fluoreszierenden Verbindung, die mindestens eine quartäre Ammoniumgruppe umfasst und frei von Carboxyl- und/oder Sulfonylgruppen ist;
- Markieren der Proben einer Faser bzw. von Fasern mit der kationischen fluoreszierenden Verbindung;
- Bewerten der Fluoreszenz jeder Probe einer Faser bzw. von Fasern mittels einer Quelle, die eine geeignete Anregungswellenlänge bzw. geeignete Anregungswellenlängen bereitstellt; und
- Vergleichen der Fluoreszenz der Proben.

3. Verfahren nach einem der vorstehenden Ansprüche, wobei die Fasern ausgewählt sind aus Säugetierhaar; vorzugsweise menschlichem Haar; mehr bevorzugt menschlichem weiblichem Haar.

4. Verfahren nach einem der vorstehenden Ansprüche, wobei die kationische fluoreszierende Verbindung ausgewählt ist aus der Gruppe von Verbindungen, die den Klassen Akridin, Azo, Diarylmethan, Eurhodin, Oxazon, Thiazol, Triarylmethan, Safranin oder Mischungen davon angehören, vorzugsweise aus der Gruppe von Verbindungen, die der Klasse Thiazol angehören.

5. Verfahren nach Anspruch 4, wobei die kationische fluoreszierende Verbindung eine Thiazolverbindung der Formel I ist:

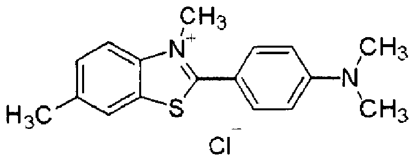

6. Verfahren nach einem der vorstehenden Ansprüche, wobei die kationische fluoreszierende Verbindung in Form einer wässrigen Lösung bereitgestellt wird, die von 1 ppm bis 3000 ppm der Verbindung, bezogen auf das Gesamtgewicht der Zusammensetzung, und einen wässrigen Träger umfasst.

7. Verfahren nach einem der vorstehenden Ansprüche, wobei die Fluoreszenz der Faser bzw. Fasern mittels eines Fluoreszenzmikroskops bewertet wird.

8. Verfahren nach Anspruch 7, wobei die Fluoreszenz der Probe qualitativ durch optische Prüfung bewertet wird und/oder wobei die Fluoreszenz der Proben quantitativ mittels einer Software-implementierten Vorrichtung bewertet wird.

9. Verfahren nach einem der vorstehenden Ansprüche, ferner umfassend den Schritt des Behandelns der Probe bzw. Proben einer Faser bzw. von Fasern mittels einer kosmetischen, chemischen und/oder mechanischen Behandlung.

10. Verfahren nach einem der Ansprüche 2 bis 9, wobei sich die Proben durch den Ursprung der Fasern, den Abschnitt der Fasern und/oder die Behandlung bzw. Behandlungen, die auf die Fasern angewendet wird bzw. werden, voneinander unterscheiden.

11. Verfahren nach Anspruch 10, wobei eine Probe eine unbehandelte Faser bzw. unbehandelte Fasern umfasst und die andere Probe eine Faser bzw. Fasern umfasst, die mit einer kosmetischen, chemischen und/oder mechanischen Zusammensetzung bzw. mit kosmetischen, chemischen und/oder mechanischen Zusammensetzungen behandelt wird bzw. werden.

12. Verfahren nach Anspruch 10, wobei die Proben mit einer unterschiedlichen kosmetischen, chemischen und/oder mechanischen Behandlung bzw. mit unterschiedlichen kosmetischen, chemischen und/oder mechanischen Behandlungen behandelt werden.

13. Verfahren nach einem der Ansprüche 9 bis 12, wobei die kosmetische Behandlung ausgewählt ist aus der Gruppe eines Shampoos, einer Konditionienmgszusammensetzung, einer Abspülbehandlung, einer zum Belassen auf dem Haar bestimmten Behandlung oder Kombinationen davon.

14. Verfahren nach einem der vorstehenden Ansprüche, ferner umfassend den Schritt des Ausspülens des Überschusses von kationischer fluoreszierender Verbindung.

15. Verfahren nach einem der vorstehenden Ansprüche, ferner umfassend den Schritt der Nutzung der Bewertung und/oder des Vergleichs zur Stützung von Werbeaussagen über die Wirksamkeit einer Behandlung gegenüber einer Kontrolle und/oder über die Überlegenheit einer Behandlung gegenüber der anderen.

## Revendications

1. Procédé pour évaluer les dommages de fibres de kératine comprenant les étapes consistant à :
- fournir au moins un échantillon de fibre(s) de kératine ;
- fournir un composé fluorescent cationique comprenant au moins un groupe ammonium quaternaire et étant exempt de groupes carboxyle et/ou sulfonyle ;
- marquer ledit échantillon de fibre(s) avec ledit composé fluorescent cationique ;
- évaluer la fluorescence de la ou des fibre(s) en utilisant une source fournissant une ou plusieurs longueur(s) d'onde d'excitation appropriée(s).

2. Procédé selon la revendication 1, pour évaluer et comparer les dommages de différentes fibres de kératine comprenant les étapes consistant à :
- fournir au moins deux échantillons différents de fibre(s) de kératine ;
- fournir un composé fluorescent cationique comprenant au moins un groupe ammonium quaternaire et étant exempt de groupes carboxyle et/ou sulfonyle ;
- marquer lesdits échantillons de fibre(s) avec ledit composé fluorescent cationique ;
- évaluer la fluorescence de chaque échantillon de fibre(s) en utilisant une source fournissant une ou plusieurs longueur(s) d'onde d'excitation appropriée(s) ; et,
- comparer la fluorescence des échantillons.

3. Procédé selon l'une quelconque des revendications précédentes, dans lequel les fibres sont choisies parmi des poils/cheveux de mammifère ; de préférence, des cheveux humains ; plus préférablement des cheveux humains féminins.

4. Procédé selon l'une quelconque des revendications précédentes, dans lequel le composé fluorescent cationique est choisi dans le groupe de composés appartenant aux classes d'acridine, azo, diarylméthane, eurhodine, oxazone, thiazole, triarylméthane, safranine ou leurs mélanges, de préférence dans le groupe de composés appartenant à la classe du thiazole.

5. Procédé selon la revendication 4, dans lequel le composé fluorescent cationique est un composé thiazole de formule I :

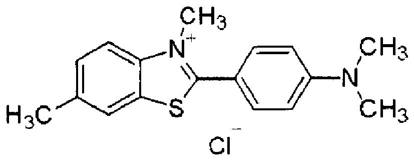

6. Procédé selon l'une quelconque des revendications précédentes, dans lequel le composé fluorescent cationique est fourni sous la forme d'une solution aqueuse comprenant de 1 ppm à 3000 ppm dudit composé en poids total de la composition et un véhicule aqueux.

7. Procédé selon l'une quelconque des revendications précédentes, dans lequel la fluorescence de la(des) fibre(s) est évaluée en utilisant un microscope à fluorescence.

8. Procédé selon la revendication 7, dans lequel la fluorescence de l'échantillon est évaluée qualitativement par inspection visuelle et/ou dans lequel la fluorescence des échantillons est évaluée quantitativement en utilisant un dispositif mis en oeuvre par logiciel.

9. Procédé selon l'une quelconque des revendications précédentes, comprenant en outre l'étape consistant à traiter le(s) échantillon(s) de fibre(s) en utilisant un traitement cosmétique, chimique et/ou mécanique.

10. Procédé selon l'une quelconque des revendications 2 à 9, dans lequel les échantillons diffèrent les uns des autres par l'origine des fibres, la partie des fibres et/ou le(s) traitement(s) appliqué(s) aux fibres.

11. Procédé selon la revendication 10, dans lequel un échantillon comprend une(des) fibre(s) non traitée(s) et l'autre échantillon comprend une(des) fibre(s) traitée(s) avec une(des) composition(s) cosmétique(s), chimique(s) et/ou mécanique(s).

12. Procédé selon la revendication 10, dans lequel les échantillons sont traités avec un (des) traitement(s) cosmétique(s), chimique(s) et/ou mécanique(s) différent(s).

13. Procédé selon l'une quelconque des revendications 9 à 12, dans lequel le traitement cosmétique est choisi dans le groupe d'un shampooing, une composition de conditionnement, un traitement à éliminer par rinçage, un traitement à conserver ou leurs combinaisons.

14. Procédé selon l'une quelconque des revendications précédentes, comprenant en outre l'étape de rinçage de l'excès de composé fluorescent cationique.

15. Procédé selon l'une quelconque des revendications précédentes, comprenant en outre l'étape d'utilisation de ladite évaluation et/ou comparaison pour soutenir des revendications publicitaires concernant l'efficacité d'un traitement par rapport à un témoin et/ou concernant la supériorité d'un traitement par rapport à l'autre.
